Europäisches Patentamt

European Patent Office

Office européen des brevets

(11) Publication number: **0 495 212 A1**

# EUROPEAN PATENT APPLICATION

(21) Application number: **91121572.1**

(22) Date of filing: **17.12.91**

(51) Int. Cl.5: **A61F 13/15**

(30) Priority: **21.12.90 JP 404330/90 U**
**19.03.91 JP 24700/91 U**

(43) Date of publication of application:
**22.07.92 Bulletin 92/30**

(84) Designated Contracting States:
**BE DE FR GB**

(71) Applicant: **NITTO DENKO CORPORATION**
**1-2, Shimohozumi 1-chome Ibaraki-shi**
**Osaka(JP)**

(72) Inventor: **Arakawa, Masaaki, c/o Nitto Denko**
**Corporation**
**1-2, Shimohozumi 1-chome**
**Ibaraki-shi, Osaka(JP)**
Inventor: **Tanaka, Naomitu, c/o Nitto Denko**
**Corporation**
**1-2, Shimohozumi 1-chome**
**Ibaraki-shi, Osaka(JP)**
Inventor: **Ohashi, Nozomu, c/o Nitto Denko**
**Corporation**
**1-2, Shimohozumi 1-chome**
**Ibaraki-shi, Osaka(JP)**

(74) Representative: **Patentanwälte Grünecker,**
**Kinkeldey, Stockmair & Partner**
**Maximilianstrasse 58**
**W-8000 München 22(DE)**

(54) **Surface material, particularly for absorbent articles.**

(57) A surface material (1) comprising one member selected from the group consisting of a nonwoven fabric (2), a foamed material (8) and a laminate of nonwoven fabric and foamed material, each having an air permeability of 5 cc/cm$^2$/sec or more and a plurality of micropores (3) having a mean pore opening of from 0.0001 to 100 mm$^2$. The material is particularly intended for use in absorbent articles such as disposable diapers, sanitary napkins, pads, towels and bandages.

FIG. 1

EP 0 495 212 A1

FIELD OF THE INVENTION

The present invention relates to a surface material for use in absorbent articles such as disposable diapers, sanitary napkins, pads, towels, and bandages. More particularly, this invention relates to a surface material which has good handling (drape characteristics), is pleasant to the skin, prevents the absorbed sweat, urine, menstrual blood, etc. from oozing out, and is air-permeable.

BACKGROUND OF THE INVENTION

Conventional surface materials for use in absorbent articles such as disposable diapers, sanitary napkins, and bandages are obtained by forming micropores in water-impermeable materials such as a polyethylene film (JP-B-57-17081). (The term "JP-B" as used herein means an "examined Japanese patent publication".)

However, since the above-described surface materials are characterized in that the water-impermeable materials used are plastic films such as polyethylene films, absorbent articles employing such a surface material are defective in that the surface material, when in contact with the skin, shows poor sweat absorption and hence the skin becomes wet with sweat, and that there are often cases where in long-term use of the absorbent article, the skin frequently develops a rash.

These drawbacks of the conventional absorbent articles are attributable to the use of water-impermeable materials as their surface materials. Although an absorbent, if present under the surface material, can absorb urine or menstrual blood, the absorptivity of the surface material itself is very low. Further, these surface materials are unpleasant to the skin. Therefore, surface materials of the above-described type are far inferior to woven surface materials.

Illustrative instances of the above are the following problems:

(1) If you holds an infant wearing a diaper employing a plastic film or sheet as a surface material, your arms and hands become wet with sweat particularly in summer;

(2) In the case of using a sanitary napkin employing a plastic film as a surface material, the skin, when in contact with the plastic film for a prolonged period of time, develops a rash in most cases and this is accelerated particularly under high-temperature and high-humidity conditions and, as a result, the skin is rubefied; and

(3) The plastic-film surface material is cold to the skin in winter.

SUMMARY OF THE INVENTION

In view of the above, the present inventors have conducted intensive studies to obtain a surface material employing a water-permeable material in place of the water-impermeable material. As a result, it has been found that the defects of the conventional absorbent articles can be greatly improved not only in water permeability but in pleasantness to the skin if a material comprising a nonwoven fabric, a foamed material, or a laminate of the nonwoven fabric and the foamed material, each having a large number of micropores, is used as a surface material. The present invention has been completed based on this finding.

Accordingly, an object of the present invention is to provide a surface material exhibiting various excellent properties when used as a surface material for absorbent articles.

The surface material in accordance with the present invention comprises one member selected from the group consisting of a nonwoven fabric, a foamed material, and a laminate of the nonwoven fabric and the foamed material, each having an air permeability of 5 $cc/cm^2/sec$ or more and a large number of micropores having a mean pore opening of from 0.0001 to 100 $mm^2$.

BRIEF DESCRIPTION OF THE DRAWINGS

Figure 1 is a perspective view of one embodiment of the surface material according to the present invention;

Figure 2 is a developmental view of a paper diaper using the surface material of the present invention;

Figure 3 is a view showing micropores in the surface material on the back side of the absorbent in the diaper of Fig. 2;

Figure 4 is a perspective view of a napkin using the surface material of the present invention;

Figures 5 (a) to (f) illustrate examples of the construction of the surface material of the present invention; and

Figures 6 (a) to (c) illustrate examples of the shape of the micropores present in the surface material of the present invention.

DETAILED DESCRIPTION OF THE INVENTION

The surface material of the present invention comprises a nonwoven fabric, a foamed material or a laminate of the nonwoven fabric and the foamed material. The nonwoven fabric, foamed material and laminate thereof each has an air permeability of 5 cc/cm$^2$/sec or more, preferably 5 to 200 cc/cm$^2$/sec, and a large number of micropores having a mean pore opening of from 0.0001 to 100 mm$^2$, preferably from 0.0001 to 50 mm$^2$.

The nonwoven fabric which can be used as the surface material of the present invention can be prepared by any conventional method. However, use of a spunbonded nonwoven fabric is particularly preferred in that fraying of fibers on the surface of the fabric is less because of the strong bonding between fibers constituting the spunbonded nonwoven fabric. Also particularly preferred as the nonwoven fabric for use in the present invention is a so-called melt-blown nonwoven fabric produced by a process in which a molten resin is blown off a multi-orifice nozzle by means of a high-temperature and high-speed gas to thereby form fibers and, at the same time, the fibers are collected on a screen. The fibers obtained are ultrafine and, hence, the nonwoven fabric obtained by the ultrafine fibers is bulky and pleasant to the skin.

The resin material for fibers of the nonwoven fabric is not particularly limited. For example, the fibers made of a polypropylene, a polyethylene, a polyester, or a polyamide, or having a core-sheath structure comprising polyethylene and a polyester (e.g., "Eleves" manufactured by Unitika, Ltd., Japan). A suitable fiber material can be selected according to the purpose of use as the surface material.

If required and necessary, the nonwoven fabric can be subjected to calender treatment or laminated with a film, thereby imparting particular properties to the nonwoven fabric according to the use of the surface material to be produced, e.g., improving the strength of the surface material.

It is also possible to conduct the calender treatment of the nonwoven fabric in a manner such that the nonwoven fabric is partly heat-treated to convert part of the surface layer to a skin layer or to partly make the surface layer to have a film-like texture. Alternatively, the entire surface can undergo such a treatment.

Examples of the foamed material which can be used in the present invention include polyolefin resin foamed materials, polyurethane resin foamed materials, and the like. Of these, polyurethane resin foamed materials are preferred from the standpoint of air permeability. The thickness of the foamed material can be freely determined according to need.

A method for forming micropores in the above-described nonwoven fabric or foamed material is a method in which the nonwoven fabric or foamed material is heat-bonded to a perforated roll having holes of the same size as that of the desired micropores and suction is conducted, or a method in which the nonwoven fabric or foamed material is pressed by a heated punching roll having a plurality of needles on the surface thereof, like a frog.

The present invention is explained by reference to the accompanying drawings.

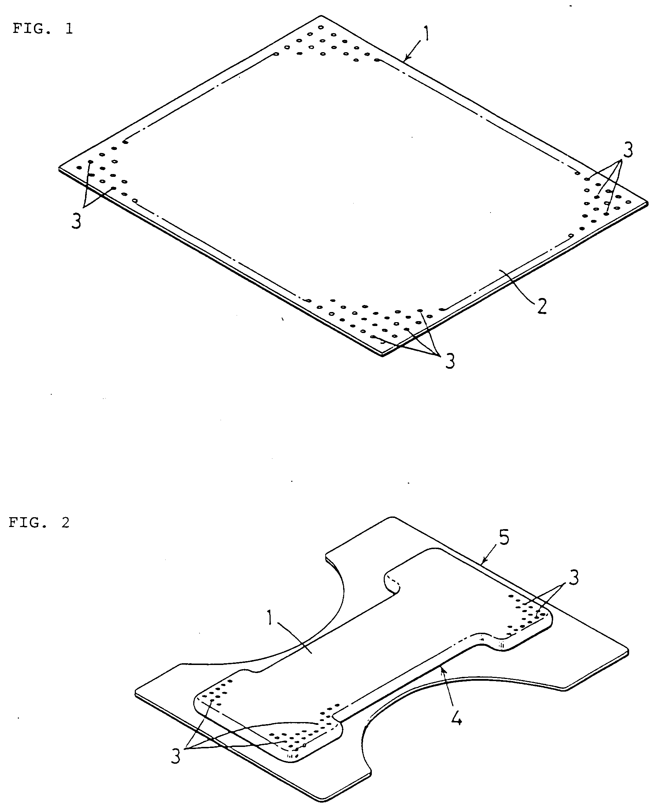

Fig. 1 is a perspective view of a surface material 1 as one example of the surface material according to the present invention. This surface material 1 has a construction comprising a nonwoven fabric 2 in which a plurality of micropores 3 have been formed.

Fig. 2 is a developmental view of a disposable paper diaper 5 which has an absorbent 4 using a surface material 1 of the present invention.

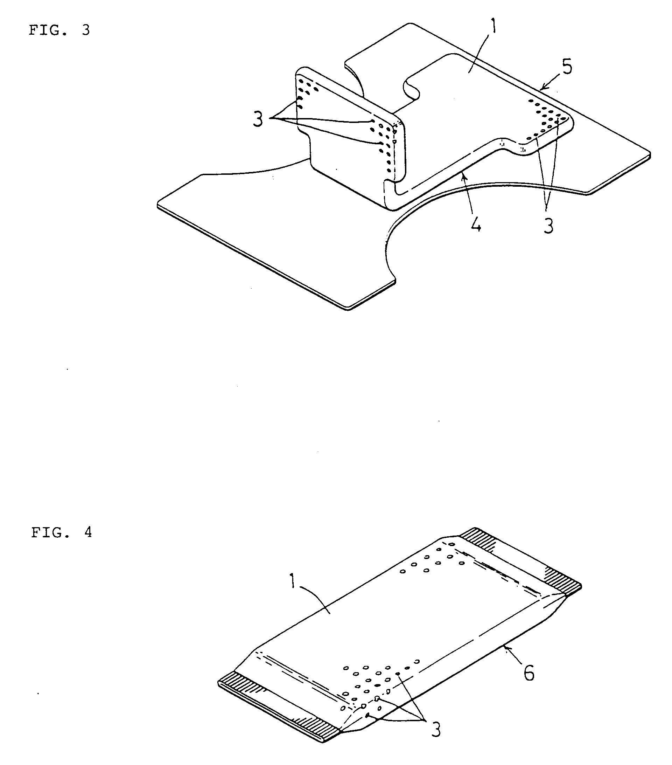

Fig. 3 illustrates micropores present in the surface material on the back side of the absorbent 4 shown in Fig. 2.

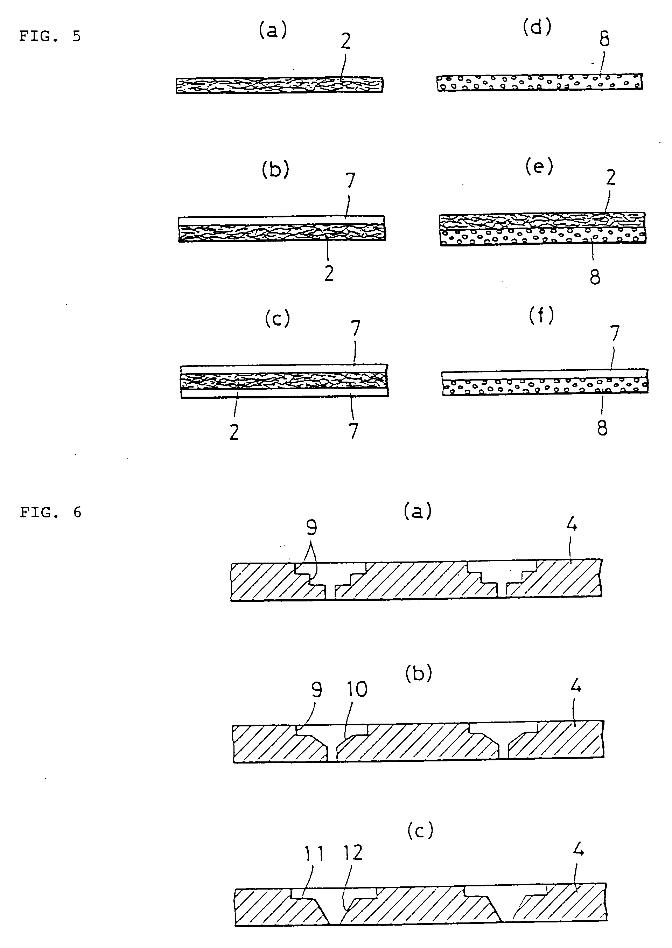

Fig. 4 is a perspective view of a napkin 6 also using a surface material 1 of the present invention.

Fig. 5 (a) to (f) show examples of the construction of the surface material 1 of the present invention, in which the surface material (a) consists of a nonwoven fabric 2 only, (b) is a laminate obtained by laminating a nonwoven fabric 2 on one side with a plastic film 7, and (c) is a laminate obtained by laminating a nonwoven fabric 2 on both sides with plastic films 7 and 7. Further, the surface material (d) consists of a foamed material 8 only, (e) is a laminate comprising a foamed material 8 and a nonwoven fabric and obtained by laminating the foamed material 8 on one side with a layer of the nonwoven fabric 2, and (f) is a laminate obtained by laminating a foamed material 8 on one side with a layer of a plastic film 7.

Fig. 6 (a) to (c) show examples of the shape of the micropores. The shape (section) of the micropores can be a stair-like structure as shown in Fig. 6 (a), a combination of a stair-like part 9 and a sloped part 10 as shown in Fig. 6 (b), or a combination of a plateau-like part 11 and a funnel-like part 12 formed from the depression of the plateau-like part 11 as shown in Fig. 6 (c). It is preferred from the standpoints of liquid absorption and prevention of the absorbed liquid from oozing out that the shape of micropores is such that the micropore becomes narrow gradually in the thickness direction from the outer side to the absorbent

EP 0 495 212 A1

side.

The characteristic feature of the present invention resides in that a plurality of micropores 3 having a mean pore opening of from 0.0001 to 100 mm$^2$ have been formed in the surface material comprising a nonwoven fabric, a foamed material, or a laminate thereof as described above. Due to these micropores, the surface material shows improved physical permeability to liquids which utilizes a capillary phenomenon.

According to the preferred embodiment of the present invention, the surface material comprises a nonwoven fabric comprising fibers of 2 deniers or finer, which has an air permeability of from 5 to 100 cc/cm$^2$/sec and a plurality of micropores having a mean pore opening of from 0.0001 to 50 mm$^2$.

The present invention will be explained in more detail by reference to the following examples, but the invention is not construed as limiting thereto.

EXAMPLE 1

In a spunbonded nonwoven fabric obtained from a polypropylene resin and having an air permeability of 20 cc/cm$^2$/sec and a basis weight of 30 g/m$^2$, micropores were formed by means of a heated roll and a heated frog. Thus, a surface material having a plurality of micropores of 0.1 mm$^2$ was obtained.

EXAMPLE 2

In a polyethylene foamed sheet having a thickness of 1,000 $\mu$m and an air permeability of 100 cc/cm$^2$/sec, micropores were formed by means of a heated roll and a heated frog. Thus, a surface material having micropores of 10 mm$^2$ was obtained.

EXAMPLE 3

A nonwoven fabric of a core-sheath structure comprising a polyethylene and a polyester ("Eleves" manufactured by Unitika, Ltd.) and having an air permeability of 20 cc/cm$^2$/sec and a basis weight of 30 g/m$^2$ was laminated with a 20 $\mu$m-thick polyethylene film. Subsequently, micropores each having several different sizes, i.e., having sizes changing stepwise from 10 mm$^2$ to 0.01 mm$^2$, were formed in the laminate by means of a frog having a plurality of needles in which the needles had sizes changing stepwise in the lengthwise direction, thereby obtaining a surface material.

Paper diapers were then prepared by replacing the surface material of the absorbent in commercially available paper diapers with each of the surface materials obtained in Examples 1 to 3 above.

COMPARATIVE EXAMPLE 1

Using a 20 $\mu$m-thick film made of low-density polyethylene, a surface material was obtained in the same manner as in Example 1. This surface material was used in place of the surface material of the absorbent in commercially available paper diapers, thereby preparing paper diapers.

The paper diapers obtained in the above Examples 1 to 3 and Comparative Example 1 were subjected to a monitor test in which the paper diapers were put on 50 infants and examined for the pleasantness of surface material to the skin, rubefaction of the skin, etc.

The results obtained are shown in Table 1.

In addition, the evaluations of the paper diapers using the surface materials of the present invention were as follows:

(1) The diapers are gentle to infants;
(2) They are easily acceptable because they are akin to cloth diapers; and
(3) They are very satisfactory because they look high-grade.

In contrast, the evaluations of the paper diapers using the surface material of Comparative Example 1 were:

(1) The surface material feels cold like films; and
(2) It is unpleasant that when the infant wearing the diaper moves, the diaper makes a rustling sound.

Thus, it was recognized that the surface materials of the present invention are excellent.

4

Table 1

| | Number of persons answering that it was pleasant to skin | Number of persons answering that the skin was wet with sweat | Number of persons answering that the diaper felt warm |
|---|---|---|---|
| Surface material of Example 1 | 49 out of 50 | 5 out of 50 | all of 50 |
| Surface material of Example 2 | 48 out of 50 | 10 out of 50 | 49 out of 50 |
| Surface material of Example 3 | 35 out of 50 | 8 out of 50 | 32 out of 50 |
| Surface material of Comparative Example 1 | 8 out of 50 | 42 out of 50 | 1 out of 50 |

EXAMPLE 4

A melt-blown nonwoven fabric formed form 0.03 denier fibers of a polypropylene resin and having an air permeability of 30 cc/cm$^2$/sec and a basis weight of 22 g/m$^2$ was brought into contact with a perforated roll having 0.1 mm$^2$ holes on its surface and then heated with suction, thereby obtaining a surface material having a plurality of 0.1 mm$^2$ pores. Paper diapers were then prepared by replacing the surface material of the absorbent in commercially available paper diapers with the thus-obtained surface material. The paper diapers were subjected to a practical use test and, as a result, good results similar to those in Examples 1 to 3 were obtained.

EXAMPLE 5

Using a melt-blown nonwoven fabric formed from 0.1 denier fibers of a polypropylene resin and having an air permeability of 40 cc/cm$^2$/sec and a basis weight of 35 g/m$^2$, a surface material having a plurality of 0.1 mm$^2$ micropores was obtained in the same manner as in Example 1.

The surface material of commercially available napkins was replaced with the surface material obtained in Example 5 above, thereby obtaining napkin samples.

COMPARATIVE EXAMPLE 2

The surface material of commercially available napkins was replaced with the surface material obtained in Comparative Example 1, thereby obtaining napkin samples.

The napkin samples obtained in the above Example 5 and Comparative Example 2 were subjected to a monitor test in which the napkins were used by 50 women and examined for the pleasantness of surface material to the skin, occurrence of a rash on the skin, etc.

The results obtained are shown in Table 2.

Table 2

| | Number of persons answering that it was pleasant to skin | Number of persons answering that the skin developed rash | Number of persons answering that the napkin gave dry feeling |
|---|---|---|---|
| Surface material of Example 4 | 47 out of 50 | 0 out of 50 | all of 50 |
| Surface material of Comparative Example 2 | 15 out of 50 | 41 out of 50 | all of 50 |

From the above, in the case of the napkins of Comparative Example 2 using the surface material of Comparative Example 1, most of the 50 persons answered that the skin had developed a rash due to plastic allergy, whereas in the case of the napkins of Example 5 using the surface material according to the present invention, good results were obtained such as (1) it was pleasant that the surface material felt clothlike and (2) the surface material was preferred in that it had improved absorbing power as compared with the film-type surface material.

Further, each of the surface materials obtained in the above Examples 1 to 4 and Comparative Example 1 was also examined for liquid absorption. In this liquid absorption test, the surface material was spread out, a few drops of water were fallen thereon from a dropper held at a height of 10 cm from the surface material, and how the water spread on the surface material was examined.

As a result, in the case of the surface materials of Examples 1 to 4, it was observed that all the water drops, which were initially in drops, spread with the lapse of time. In contrast, the water on the surface material of Comparative Example 1 remained in the form of drops.

As described above, since the surface material of the present invention comprises a nonwoven fabric, a foamed material, or a laminate thereof, each having an air permeability of 5 $cc/cm^2/sec$ or more and a plurality of micropores having a mean pore opening of from 0.0001 to 100 $mm^2$, it is completely free of the cold plastic feeling of conventional surface materials, is pleasant to the skin, and does not cause the skin to be rubefied or develop a rash. Therefore, the surface material of the present invention is practically useful not only in paper diapers but also as the surface materials of other disposable absorbent articles including napkins.

While the invention has been described in detail and with reference to specific embodiments thereof, it will be apparent to one skilled in the art that various changes and modifications can be made therein without departing from the spirit and scope thereof.

## Claims

1. A surface material comprising one member selected from the group consisting of a nonwoven fabric, a foamed material and a laminate of the nonwoven fabric and the foamed material, each having an air permeability of 5 $cc/cm^2/sec$ or more and a plurality of micropores having a mean pore opening of from 0.0001 to 100 $mm^2$.

2. A surface material as claimed in claim 1, wherein said nonwoven fabric is a spunbonded nonwoven fabric.

3. A surface material as claimed in claim 1, wherein said nonwoven fabric is a melt-blown nonwoven fabric.

4. A surface material comprising a nonwoven fabric which comprises fibers of 2 deniers or fiber and has an air permeability of from 5 to 100 $cc/cm^2/sec$ and a plurality of micropores having a mean pore opening of from 0.0001 to 50 $mm^2$.

5. A surface material as claimed in claim 4, wherein said nonwoven fabric is a spunbonded nonwoven fabric.

6. A surface material as claimed in claim 4, wherein said nonwoven fabric is a melt-blown nonwoven fabric.

FIG. 1

FIG. 2

FIG. 3

FIG. 4

FIG. 5

(a)                    (d)

(b)                    (e)

(c)                    (f)

FIG. 6

(a)

(b)

(c)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 338 479 (GESSNER)<br>* Claims 1-2,5-7 *<br>--- | 1-6 | A 61 F 13/15 |
| X | US-A-4 482 603 (T. YOSHIDA et al.)<br>* Column 7, lines 8-11,42-55 *<br>--- | 1,4 | |
| A | EP-A-0 293 482 (UNI-CHARM)<br>* Abstract; page 8, line 26 - page 9, line 9; page 13, lines 8-12; page 14, lines 25-26; tables 1-2 *<br>--- | 1-6 | |
| A | EP-A-0 286 543 (KIMBERLEY-CLARK)<br>* Column 1, lines 27-33; column 2, lines 5-23 *<br>--- | 1-2,4-5 | |
| A | DE-A-2 049 978 (DEUTSCHE SEMPERIT GUMMIWERK)<br>* Claims 1,3,6,12 *<br>----- | 1-2,5 | |
|  |  |  | TECHNICAL FIELDS SEARCHED (Int. Cl.5)<br><br>A 61 F |

The present search report has been drawn up for all claims

| Place of search | Date of completion of the search | Examiner |
|---|---|---|
| THE HAGUE | 25-03-1992 | NICE P.R. |

EPO FORM 1503 03.82 (P0401)